Europäisches Patentamt

European Patent Office

Office européen des brevets

(19)

(11) Publication number: 0 302 459
A2

## (12) EUROPEAN PATENT APPLICATION

(21) Application number: 88112573.6

(22) Date of filing: 02.08.88

(51) Int. Cl.⁴: A61K 35/50 , A61K 37/02

(30) Priority: 04.08.87 JP 193792/87

(43) Date of publication of application:
08.02.89 Bulletin 89/06

(84) Designated Contracting States:
BE CH DE FR GB LI NL SE

(71) Applicant: SUMITOMO PHARMACEUTICALS
COMPANY, LIMITED
40, Dosho-machi 2-chome
Higashi-ku Osaka(JP)

Applicant: MEGURO INSTITUTE CO., LTD.
7-29, Masumicho
Ikeda-shi Osaka(JP)

(72) Inventor: Ueba, Keizo
6-44, Fushiodai-2-chome
Ikeda-shi(JP)
Inventor: Higo, Shinji
1-4, Kamihozumi-2-chome
Ibaraki-shi(JP)
Inventor: Ozawa, Kyosuke
3-25, Seiwadaihigashi-5-chome
Kawanishi-shi(JP)

(74) Representative: Henkel, Feiler, Hänzel &
Partner
Möhlstrasse 37
D-8000 München 80(DE)

(54) Human placenta-derived thermostable cell growth factor and a process for production thereof.

(57) By extraction-treating human placenta with water or an acidic medium with heating at 80°C to 120°C, isolating a protein fraction from the extract and fractionating the protein fraction using ion exchange resin, a human placenta-derived, thermostable low molecular cell growth factor having a cell growth-stimulating activity on animal cells and having excellent safety is obtained.

EP 0 302 459 A2

# HUMAN PLACENTA-DERIVED THERMOSTABLE CELL GROWTH FACTOR AND A PROCESS FOR PRODUCTION THEREOF

BACKGROUND OF THE INVENTION

## 1. FIELD OF THE INVENTION

The present invention relates to a cell growth factor having excellent thermostability which is obtained from human placenta and a process for production thereof.

## 2. DESCRIPTION OF THE RELATED ART

In recent years, the rapid progress and spread of genetic technology have enabled mass production of various physiologically active substances in vitro which had been hitherto produced only in vivo.

At present, cells of microorganism such as bacteria, yeast or the like which can be readily cultivated in large scale have been generally utilized in the case that the objective physiologically active substance is produced by means of genetic technology. It is known, however, that the physiologically active substance produced utilizing microorganism differs from one produced from animal tissue in some delicate point.

Therefore, in order to produce physiologically active substances that are truly useful physiologically or pharmacologically, it becomes necessary to culture animal-, in particular, human-derived cells in large scale and whereby to produce physiologically active substances.

However, serum of good quality such as bovine fetal serum, etc. must be supplemented to a medium upon culture of animal cells, and such circumstance results in not a few obstacles in the production of physiologically active substances by culture of animal cells. Namely, in serum, its components or physiological activity greatly vary among animal individuals so that there is a problem that constant serum-containing medium is not always prepared. Further, animal serum might be sometimes contaminated with various microorganisms, in particular, viruses or mycoplasma, thus to denature and kill culture cells. In addition, viruses or mycoplasma might be intermingled with the produced substance in some occasion to cause serious results.

The most serious problem caused by supplementation of serum is that upon isolation and purification of the objective physiologically active substance from the culture solution, it is practically difficult to completely remove the serum component used; even though it is possible to remove the serum component, this operation is not preferred from an economical viewpoint.

In view of the foregoing circumstances, various so-called serum-free media composed only of components having known chemical properties have been developed heretofore to overcome the problem in animal cell culture caused by the addition of serum.

In these medium, however, protein components derived from animals other than human such as bovine serum albumin or decomposition products thereof, bovine insulin and other various growth factors, etc. are contained.

Recently, there have been proposed methods for isolating a cell growth factor or a cell growth stimulator from human placenta (Japanese Patent Application KOKAI (Laid-Open) Nos. 61-229894 and 62-84025, etc.). These methods comprise extraction-treating human placenta at room temperature or at low temperature, so that it is impossible to fully inactivate microorganisms and toxic substances. In addition, the thus obtained substances have high molecular weights and are effective only for growth of specific cells. That is, human placenta is always contaminated with many bacteria, especially, intestinal bacteria that are always present there. Furthermore, some of the sera might suffer from infections via placenta with AIDS virus (HIV), cytomegalovirus, etc. and from contamination at the birth canal with hepatitis B virus (HBV). Accordingly, it should be considered that human placenta materials are always contaminated with bacteria or toxins (especially endotoxin) and further with pathogenic viruses, etc.

With respect to substances obtained as described above, in the case that these substances have high molecular weights, there is a problem of inducing allergic reaction when they are applied to the living body even though they are derived from human. In addition, the heat treatment is inadequate and therefore, there is also a problem that it is undesired to utilize them in serum-free medium.

## SUMMARY OF THE INVENTION

Accordingly, an object of the present invention is to provide a cell growth factor having a low molecular weight which is effective for culture of animal cells in serum-free medium, thermostable, safe and excellent in stability.

Another object of the present invention is to provide a process for production of the cell growth factor.

One aspect of the present invention is directed to a human placenta-derived thermostable cell growth factor, which is prepared from a fraction incapable of forming precipitates with ammonium sulfate in a concentration of at 40% saturation obtained from human placenta, comprising as main ingredient a peptide having a molecular weight of about 450 or a polymer thereof and/or a peptide having a molecular weight of about 1,300, and having properties showing resistance to temperature of 120°C and weak affinity to an ion exchange resin.

Another aspect of the present invention is directed to a method for a human placenta-derived thermostable cell growth factor, which comprises extraction-treating human placenta with water or an acidic medium, isolating a protein fraction from the extract and fractionating the protein fraction by means of ion exchange resin thereby to prepare the human placenta-derived thermostable cell growth factor mentioned above.

## BRIEF DESCRIPTION OF THE DRAWINGS

Fig. 1 shows 5 peaks appearing in an eluate obtained by developing a partially purified solution through a DEAE-cellulose column, which is obtained in Example 1.

Fig. 2 shows two peaks appearing in an eluate obtained by developing Fraction II of Fig. 1 through a Sephadex G-50 column.

Figs. 3 and 4 show the single peak when the two peaks appearing in the eluate from Fraction II are individually subjected to high performance liquid chromatography and eluted with 0.05% TFA.

Fig. 5 shows the single peak when Fraction III in Fig. 1 is developed through Sephadex G-50.

Fig. 6 shows the single peak when the single peak in Fig. 5 is subjected to high performance liquid chromatography.

## DETAILED DESCRIPTION OF THE PREFERRED EMBODIMENTS

The human placenta used as the staring material in the present invention is preferably placenta obtained by freeze-treating normally delivered human placenta at -20°C for several days and then homogenizing the resulted placenta.

The thus obtained human placenta is firstly subjected to an extraction treatment. The extraction treatment is performed by adding an acidic medium such as an acetate buffer, etc. to the homogenized placenta to adjust pH value to about 4.0 and stirring at temperature of 80°C for 2 hours or longer. In this case, water may also be used as a medium for the extraction. According to the present invention, the extraction is carried out with heating at temperatures of 80°C to 120°C so that contaminanated bacteria or viruses present in the placenta can be sterilized. Thus, disturbances which might be caused by the contamination of human placenta as described above can be eliminated by the present invention. When the heating is carried at a temperature higher than 120°C, the objective growth factor obtained by the extraction is adversely affected and such heating is not preferred. With temperatures lower than 80°C, the sterilizing effect is not sufficiently achieved and such heating is not preferred.

Then, a protein fraction is isolated from the thus obtained extract. The isolation of protein fraction is preferably conducted as follows. After cooling, the supernatant of the extract is collected and a several-fold amount of cold ethanol is added to the supernatant. The mixture is stirred at about 0°C. Then, the formed insoluble materials are removed by centrifugation, etc. The obtained supernatant is dried and then dissolved in physiological saline to prepare a crude fraction of the growth factor according to the present invention.

In the treatment of the extract described above, the isolation of protein fraction may also be performed by means of precipitation with sulfate ammonium instead of the isolation using cold ethanol. Namely, after cooling the extract, the extract is centrifuged and the supernatant is collected. Ammonium sulfate is added to the supernatant to make its concentration at 40% saturation. The thus obtained supernatant may also be used as the crude fraction of the growth factor according to the present invention.

The crude fraction is subjected to column chromatography using an ion exchange resin such as DEAE

3

cellulose, etc. to bring into contact with the ion exchange resin. Then, the active fraction exerting a cell growth-stimulating activity is eluted with, for example, 50 mM Tris-hydrochloride buffer (pH 6.5). The thus obtained active fraction is further subjected to gel filtration on Sephadex, etc. and/or high performance liquid chromatography, thereby to purify the active fraction to a higher purity.

With the thus obtained cell growth-stimulating activity fraction, it is confirmed that the fraction contains as main ingredient a peptide having a molecular weight of about 450 and a polymer thereof and/or a peptide having a molecular weight of about 1,300, which were determined by Sephadex gel filtration. The molecular weight of the polymer of the peptide having a molecular weight of about 450 is about 9,600 as will be evident from the examples later described.

In the present invention, the thus obtained active fraction is utilized as the cell growth factor. The cell growth factor according to the present invention does not form precipitates with ammonium sulfate at a concentration of at 40% saturation, more specifically, the peptide having a molecular weight of about 450 or the polymer thereof is capable of forming precipitates with ammonium sulfate in a concentration of at 80% saturation and the peptide having a molecular weight of about 1,300 is incapable of forming precipitates at the same concentration, and further the cell growth factor has the following properties.

i) The cell growth factor shows resistance to temperature of 120 °C.

ii) The cell growth factor shows weak affinity to an ion exchange resin.

The cell growth factor further posseses such property that the factor does not form precipitates by a treatment with cold ethanol, as is clear from the treatment of the extract described above.

With regard to the use of the cell growth factor of the present invention, the growth factor may be added in an appropriate quantity (for example, 5 to 15%) to a various synthetic medium (Eagle's MEM, Dulbecco's modified MEM, RPMI 1640, etc.), whereby the growth factor can proliferate mouse hydridomas 3Flb and KJ-01, animal cells such as Rhesus monkey kidney cell MA-104, etc. to the same extent as in or more than in medium supplemented with bovine fatal serum.

In some occasion, the cell growth-stimulating effect of the growth factor according to the present invention may not be sufficient depending upon kind of cells and combination with synthetic culture solution (basal medium); in such a case, however, an extremely high cell growth-stimulating effect can be obtained by incorporating a trace amount of fibronectin, transferrin, or, if necessary and desired, gelatin, etc.

The cell growth factor according to the present invention has excellent thermostability as described above. Therefore, it is possible to sterilize the cell growth factor under high pressure upon its preparation. Thus, according to the cell growth factor of the present invention, animal cells can be cultured safely, which serves to improvement in productivity of the physiologically active substance by mass production.

Hereafter the present invention and its effects will be concretely described by referring to the examples below.

Example 1

Normally delivered human placenta, 50 g, which had been stored by freezing, was minced and 50 ml of 50 mM sodium acetate buffer was added thereto. While stirring at a temperature of 80 °C for 2 hours, extraction was performed. The obtained extract was subjected to centrifugation (for one hour at 3000 r.p.m.) to give the supernatant. A 6-fold amount of chilled ethanol was added to the supernatant. After stirring at 4 °C for 30 minutes, the supernatant was separated by centrifugation.

After the obtained supernatant was evaporated off to dryness with a rotary evaporator, the residue was dissolved in 12.5 ml of physiological saline. The solution was further subjected to centrifugation to give the supernatant. This supernatant was used as a partially purified solution. The partially purified solution was subjected to column chromatography on DEAE-cellulose column (3.2 x 50 cm) which had been previously equilibrated with 50 mM Tris-hydrochloride buffer (pH 6.5), followed by eluting with the same buffer as used for the equilibration.

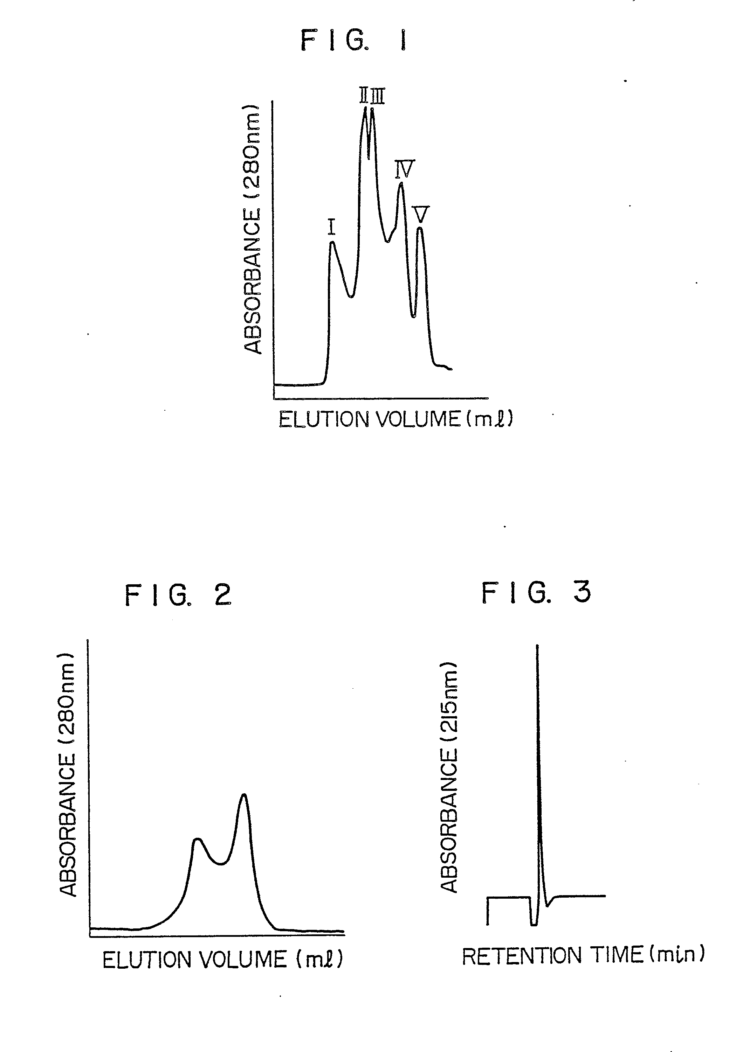

In the eluate obtained, 5 peaks appeared as shown in Fig. 1. The cell growth-stimulating activity was observed in the second fraction (Fraction II, 60 ml, absorbancy at 280 nm = 0.9635) and the third fraction (Fraction III, 66 ml absorbancy at 280 nm = 1.8850), as shown in Table 1. The cell growth-stimulating activity was determined according to the method of Yabe (Yabe, N., et al., In Vitro Cellular and Development Biology, 21:815-820, 1987).

Table 1

| Test Cell | Fraction | | | | |
|---|---|---|---|---|---|
| | I | II | III | IV | V |
| 3F1b[a] | - | + | + | - | - |
| KJ-01[b] | - | + | + | - | - |
| MA-104[c] | - | + | + | - | - |
| (Notes) + ... Positive in the cell growth-stimulating activity | | | | | |
| - ... Negative in the cell growth-stimulating activity | | | | | |

a ... Anti-HBe antibody-producing mouse hydridoma
b ... Anti-CRP antibody-producing mouse hydridoma
c ... Rhesus monkey kidney cell line

Fraction II described above was subjected to column chromatography on Sephadex G-50 column (2.2 x 100 cm) equilibrated with a buffer containing 50 mM Tris-hydrochloride, 0.15 M sodium chloride and 5 mM EDTA (pH 6.5). Elution was carried out with the same buffer as used for the equilibration. Two peaks were recognized in the eluate as shown in Fig. 2. The molecular weights of the peaks were about 450 and 9,600, respectively.

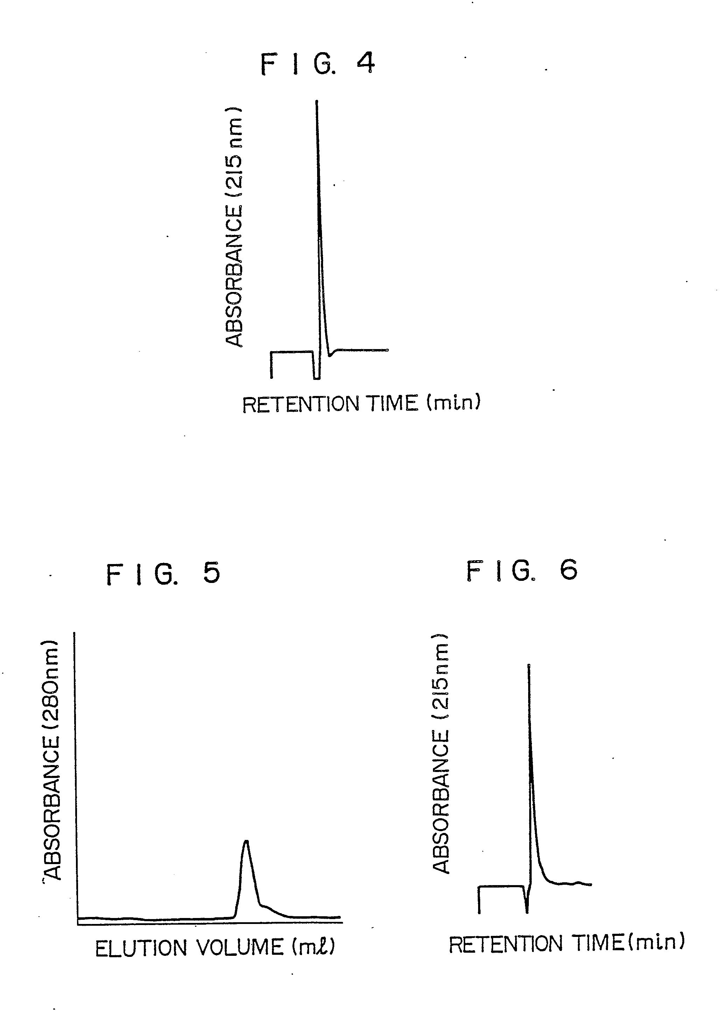

Each of these peaks was subjected to high performance liquid chromatography on ZORBAX ODS column (0.4 x 25 cm), followed by elution with 0.05% trifluoroacetic acid (TFA) (ml/min.) Both peaks showed a individual single peak (Figs. 3 and 4), which retention time was identical with each other and 3.20 minutes in both.

That is, it was recognized that the two peaks described above comprised a peptide having a molecular weight of about 450 and its polymer.

Fraction III described above was subjected to column chromatography on Sephadex G-50 in a similar manner, resulted in the single peak as in Fig. 5. Its molecular weight was about 1,300. In a similar manner, high performance liquid chromatography of the peak showed the single peak at retention time of 3.28 minutes as shown in Fig. 6.

When the amounts of proteins in Fraction II and Fraction III were assayed by the Lowry method, absorbance coefficient $E_{1\%}^{280}$ was 9.04 (Fraction II) and 22.05 (Fraction III), respectively.

Example 2

After the partially purified solution obtained in Example 1 was heated at 120°C for 10 minutes, the solution was centrifuged and ammonium sulfate was added to the resulting supernatant to make 40% saturation. After stirring at 4°C for an hour, centrifugation was conducted to give the supernatant. Ammonium sulfate was further added to the supernatant to make 80% saturation. After stirring, the precipitate and supernatant were obtained by centrifugation.

The precipitate was dissolved in 50 mM Tris-hydrochloride buffer (pH 6.5) and the solution was developed through a DEAE-cellulose column in a manner similar to Example 1. Then, the major peak was subjected to high performance liquid chromatography to give a single peak. The retention time in this case showed 3.20 minutes which was the same as in Fraction II described above.

On the other hand, the supernatant was developed through a DEAE-cellulose column and subjected to high performance liquid chromatography, whereby the single peak appeared and its retention time was 3.28 minutes which was the same as Fraction III described above.

While the invention has been described in detail and with reference to specific embodiments thereof, it is apparent to one skilled in the art that various changes and modifications scope of the present invention.

## Claims

1. A human placenta-derived thermostable cell growth factor, which is prepared from a fraction incapable of forming precipitates with ammonium sulfate in a concentration of at 40% saturation obtained from human placenta, comprising as main ingredient a peptide having a molecular weight of about 450 or a polymer thereof and/or a peptide having a molecular weight of about 1,300, and having properties showing resistance to temperature of 120° C and weak affinity to an ion exchange resin.

2. A human placenta-derived thermostable cell growth factor according to claim 1, wherein the peptide having a molecular weight of about 450 or the polymer thereof is capable of forming precipitates with ammonium sulfate in a concentration of at 80% saturation.

3. A human placenta-derived thermostable cell growth factor according to claim 1, wherein the peptide having a molecular weight of about 1,300 is incapable of forming precipitate with ammonium sulfate in a concentration of at 80% saturation.

4. A human placenta-derived thermostable cell growth factor according to claim 1, which exerts a cell growth-stimulating activity on animal cells.

5. A human placenta-derived thermostable cell growth factor according to claim 1, which is incapable of forming precipitates with cold ethanol.

6. A process for producing a human placenta-derived thermostable cell growth factor, which comprises extrac tion-treating human placenta with water or an acidic medium, isolating a protein fraction from the extract and fractionating the protein fraction by means of ion exchange resin thereby to prepare a human placenta-derived thermostable cell growth factor of claim 1.

7. A process according to claim 6, wherein said extraction is conducted with heating at a temperature of 80 to 120° C.

8. A process according to claim 6, wherein isolation of said protein fraction from the extract is conducted by a treatment with cold ethanol or a precipitation with ammonium sulfate.

9. A process according to claim 6, wherein isolation of said protein fraction from the extract is conducted by the precipitation with ammonium sulfate in a concentration of at 40% saturation.

10. A process according to claim 6, wherein said protein fraction is further fractionated by gel filtration and/or high performance liquid chromatography.

# F I G. 1

# F I G. 2

# F I G. 3

# F I G. 4

ABSORBANCE (215 nm)

RETENTION TIME (min)

# F I G. 5

ABSORBANCE (280nm)

ELUTION VOLUME (ml)

# F I G. 6

ABSORBANCE (215nm)

RETENTION TIME (min)